(19) Europäisches Patentamt
European Patent Office
Office européen des brevets

(11) **EP 3 782 632 A1**

(12) **EUROPEAN PATENT APPLICATION**
published in accordance with Art. 153(4) EPC

(43) Date of publication:
**24.02.2021 Bulletin 2021/08**

(21) Application number: **19778028.1**

(22) Date of filing: **25.03.2019**

(51) Int Cl.:
*A61K 35/747* (2015.01)  *A23L 33/135* (2016.01)
*A61P 25/00* (2006.01)  *C12N 1/20* (2006.01)

(86) International application number:
**PCT/JP2019/012303**

(87) International publication number:
**WO 2019/188868 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2018  JP 2018060637**

(83) **Declaration under Rule 32(1) EPC (expert solution)**

(71) Applicant: **Morinaga Milk Industry Co., Ltd.
Minato-ku
Tokyo 108-8384 (JP)**

(72) Inventors:
• **KOBAYASHI Yodai
Zama-shi, Kanagawa 252-8583 (JP)**
• **MAEHATA Hazuki
Zama-shi, Kanagawa 252-8583 (JP)**
• **KUHARA Tetsuya
Zama-shi, Kanagawa 252-8583 (JP)**
• **TOYODA Atsushi
Inashiki-gun, Ibaraki 300-0393 (JP)**

(74) Representative: **Vossius & Partner
Patentanwälte Rechtsanwälte mbB
Siebertstrasse 3
81675 München (DE)**

(54) **ANTI-STRESS COMPOSITION**

(57) An object is to provide an anti-stress composition that can be administered for a long period of time and is highly safe. Provided is an anti-stress composition including *Lactobacillus helveticus* as an active ingredient. The stress may be mental stress. Furthermore, the composition may be used for treatment or prevention of anxiety disorder, anthropophobia, a decline in sociality, or adjustment disorder. Furthermore, the *Lactobacillus helveticus* may be *Lactobacillus helveticus* NITE BP-01671. Furthermore, the composition may be a pharmaceutical composition or a food or drink composition.

EP 3 782 632 A1

**Description**

Technical Field

The present technology relates to an anti-stress composition including *Lactobacillus helveticus* as an active ingredient.

Background Art

In modern stressful society, people are exposed to various stresses due to changes in the living environment and the working environment, complicated human relations, physical and mental fatigue, and the like.
In order to relieve such stresses, various anti-stress agents have been developed. For example, there is known an alleviating or preventing agent for stress symptoms, the agent including an oxidized coenzyme and/or a reduced coenzyme as an active ingredient (Patent Document 1).

Citation List

Patent Document

Patent Document 1: JP-A-2010-030901

Summary of the Invention

Technical Problem

Since people are subjected to such stresses on a daily basis, it is preferable that the anti-stress agent can be administered for a long period of time and is highly safe.
Thus, a main object of the technology is to provide an anti-stress composition that can be administered for a long period of time and is highly safe.

Solution to Problem

In recent years, due to the complication of the social structure and human relationship caused by acts such as power harassment or sexual harassment by a strong person, the stress that a vulnerable person is subjected to has emerged as a new social problem. Although it is desired to reduce such stress, for the present, sufficient knowledge has not been obtained.
Thus, the inventors of the technology established an animal model for evaluating the stress that a vulnerable person is subjected to by an attack from a strong person and conducted a thorough investigation. The inventors found that when *Lactobacillus helveticus* is administered, there is an anti-stress effect, specifically, an effect of reducing mental stress.
That is, the technology is as follows.

[1] An anti-stress composition, comprising *Lactobacillus helveticus* as an active ingredient.
[2] The composition according to the above-described item [1], in which the stress is mental stress.
[3] The composition according to the above-described item [1] or [2], in which the composition is used for treatment or prevention of anxiety disorder, anthropophobia, a decline in sociality, or adjustment disorder.
[4] The composition according to any one of the above-described items [1] to [3], in which the *Lactobacillus helveticus* is *Lactobacillus helveticus* NITE BP-01671.
[5] The composition according to any one of the above-described items [1] to [4], in which the composition is a pharmaceutical composition or a food or drink composition.

Advantageous Effects of the Invention

According to the technology, an anti-stress composition that exhibits reduced adverse side effects, can be administered for a long period of time, and is highly safe, can be provided. Meanwhile, the effect of the technology is not necessarily limited to the effects described herein and may be any one of the effects described in the present specification.

Description of Embodiments

Preferred embodiments of the technology will be described below. However, the technology is not limited to the following embodiments and can be freely modified within the scope of the technology. Meanwhile, unless particularly stated otherwise, the percentage used in the present specification is percentage based on mass.

The technology can provide an anti-stress composition including *Lactobacillus helveticus* as an active ingredient.

Regarding the bacterium that is classified as *Lactobacillus helveticus* of the technology, among the bacteria belonging to the genus *Lactobacillus,* any lactic acid bacterium that belongs to the species *Lactobacillus helveticus,* which includes bacteria that are classified as *helveticus,* can be used.

Among them, it is preferable to use *Lactobacillus helveticus* NITE BP-01671.

*Lactobacillus helveticus* NITE BP-01671 was deposited on July 29, 2013, to National Institute of Technology and Evaluation Patent Microorganisms Depositary (Room No. 122, 2-5-8, Kazusakamatari, Kisarazu-shi, Chiba Prefecture, 292-0818, Japan), as *Lactobacillus helveticus* MCC1848 (Deposit No.: NITE BP-01671) . This bacterium can be generally purchased from the above-described depositary.

*Lactobacillus helveticus* NITE BP-01671 is not limited to the deposited bacterium, and may be a bacterium that is substantially homogeneous with the same deposited bacterium. A substantially homogeneous bacterium is a bacterium that is classified as *Lactobacillus helveticus,* the base sequence of 16SrRNA gene of which is at least 99.5% identical, and preferably 100% identical, to the deposited bacterium, or which has the same microbiological characteristics as those of the above-described deposited bacterium, and a strain that is substantially identical to the above-described deposited strain is also included (JP-A-2015-047114).

With regard to the bacterial strain, a "strain that is substantially equivalent to the above-described deposited strain" means a strain which belongs to the same species as the deposited strain, and from which an anti-stress effect as an effect of the technology is obtained. The strain that is substantially equivalent to the deposited strain may be, for example, a derivative strain produced from the deposited strain as a parent strain. Examples of the derivative strain include a strain bred from the deposited strain, and a strain spontaneously produced from the deposited strain.

Examples of the substantially same bacterial strain and the derivative strain include the following strains:

(1) A bacterial strain determined to be the same bacterial strain as *Lactobacillus helveticus* NITE BP-01671 by a randomly amplified polymorphic DNA (RAPD) method or a pulsed-field gel electrophoresis method (PFGE method) (described in Probiotics in food/Health and nutritional properties and guidelines for evaluation 85, Page 43).

(2) A bacterial strain having at least 95% DNA identity with *Lactobacillus helveticus* NITE BP-01671, the bacterial strain having *Lactobacillus helveticus* NITE BP-01671-derived genes only but not having foreign-derived genes.

(3) A strain bred from *Lactobacillus helveticus* NITE BP-01671 (including strains bred by genetically engineered modification, mutation, and spontaneous mutation), the strain having the phenotype of NITE BP-01671.

*Lactobacillus helveticus* of the technology can be proliferated by, for example, culturing. A method for culturing is not particularly limited as long as *Lactobacillus helveticus* of the technology can be proliferated thereby, and any method that is conventionally used for the culture of lactic acid bacteria can be used, after being appropriately modified as necessary. For example, the culturing temperature may be 25°C to 45°C, and is preferably 30°C to 42°C, and particularly preferably 37°C to 42°C. Furthermore, it is preferable that culture is carried out under anaerobic conditions, and for example, culture can be carried out under ventilation with an anaerobic gas such as carbon dioxide. Furthermore, culture may also be carried out under slightly aerobic conditions such as liquid stationary culture.

Regarding a medium that is used for the culture for proliferating *Lactobacillus helveticus* of the technology, for example, a medium that is conventionally used for the culture of lactic acid bacteria can be used, after being appropriately modified as necessary. That is, regarding a carbon source, for example, saccharides such as galactose, glucose, fructose, mannose, cellobiose, maltose, lactose, sucrose, trehalose, starch, starch hydrolysate, and molasses can be used depending on assimilability. Regarding a nitrogen source, for example, ammonia; ammonium salts such as ammonium sulfate, ammonium chloride, and ammonium nitrate; and nitric acid salts can be used. Furthermore, regarding an inorganic salt, for example, sodium chloride, potassium chloride, potassium sulfate, magnesium sulfate, calcium chloride, calcium nitrate, manganese chloride, and ferrous sulfate can be used. In addition, organic components such as peptone, soybean flour, defatted soybean cake, meat extract, and yeast extract may also be used. Furthermore, regarding a medium that is conventionally used for the culture of bacteria of the genus *Bifidobacterium,* specifically, reinforced clostridial medium, MRS medium (de Man, Rogosa, and Sharpe medium), mMRS medium (modified MRS medium), TOS propionate medium (TOSP medium), and TOS propionate mupirocin medium (TOSP Mup medium) may be mentioned.

As the *Lactobacillus helveticus* of the technology, after culture, a culture product thus obtained may be directly used, the culture product may be used after being diluted or concentrated, or bacterial cells collected from the culture product may be used. Furthermore, as long as the effects of the technology are not impaired, various additional operations such as heating and freeze-drying can be carried out after culturing. Furthermore, the present bacterial cells may be viable bacteria or killed bacteria; however, it is preferable that the bacterial cells are killed bacteria, and regarding the killed bacteria, killed bacteria that have been sterilized by heating, freeze-drying, or the like may be mentioned. Examples of other methods for preparing killed bacterial cells include a spray drying method (spray-dry method), a retort sterilization method, a freeze-drying method, a UHT sterilization method, a pressure sterilization method, a high-pressure steam sterilization method, a dry heat sterilization method, a flowing steam disinfection method, an electromagnetic wave sterilization method, an electron beam sterilization method, a high-frequency sterilization method, a radiation sterilization method, an ultraviolet radiation sterilization method, an ethylene oxide gas sterilization method, a hydrogen peroxide gas plasma sterilization method, and chemical sterilization methods (an alcohol sterilization method, a formalin fixation method, and an electrolytic water treatment method). Furthermore, the present bacterial cells may be in the form of crushed material. The crushed material may be a material obtained by crushing viable bacteria or may be a material obtained by crushing killed bacteria, and a material that has been subjected to heating, freeze-drying, or the like after crushing may also be used.

With regard to the anti-stress composition of the technology, since the active ingredient is *Lactobacillus helveticus,* which is a lactic acid bacterium, the anti-stress composition is excellent in regard to safety, and so there is little need to worry about adverse side effects even if the anti-stress composition is administered continuously for a long period of time, and therefore, the anti-stress composition is very useful. Furthermore, the anti-stress composition is highly safe even for use in combination with another composition.

As shown in the Examples that will be described below, *Lactobacillus helveticus* of the technology has an anti-stress effect, specifically an effect of mitigating mental stress, and more specifically an effect of mitigating interpersonal stress (for example, social defeat stress) . Therefore, *Lactobacillus helveticus* of the technology can be used as an anti-stress composition. Furthermore, *Lactobacillus helveticus* can be used for a variety of use applications such as pharmaceutical products and foods, or may be a material or a preparation, which is incorporated into these and used.

The term "anti-stress" according to the technology means favorable turn, prevention of aggravation, delay, inversion of progress, treatment, or the like of a symptom or a disease caused by stress. Furthermore, the "anti-stress" according to the technology also includes the meaning of prevention of a disease that develops as a result of stress. Meanwhile, the term "prevention" according to the technology means prevention of onset or delay of onset of a symptom or a disease in a subject of application, or a decline in the danger of onset of a symptom or a disease in a subject of application, or the like.

Furthermore, the "stress" according to the technology includes physical stress, chemical stress, mental stress, and the like. According to the technology, the "stress" is preferably mental stress.

The "mental stress" according to the technology means a situation in which mental stress is felt under particular circumstances, and thus psychiatric symptoms, somatic symptoms, and behavioral symptoms are manifested. As the symptoms, a depressed mood, anxiety, a decline in enthusiasm or concentration power, a feeling of irritation, and the like are shown, and as somatic symptoms, headache, dizziness, palpitation, feeling of fatigue, and the like are shown.

The anti-stress composition of the technology is used preferably for the prevention, reduction, or suppression of stresses that a person is subjected to because of a change in the environment or interpersonal relations. Furthermore, the composition of the technology can be preferably used for the treatment or prevention of a disease caused by stresses that a person is subjected to for a medium to long period of time because of a change in the environment, specifically, for the treatment or prevention of anxiety disorder, anthropophobia, a decline in sociality, or adjustment disorder.

With regard to the anti-stress composition of the technology, specifically a patient who has developed the following stress diseases can be a subject of administration:

1) Depression, anxiety disorder, anthropophobia, a decline in sociality, and adjustment disorder that are suffered by a person of lower social status who chronically receives aggressive language or intimidating attitude from a person of higher social status over a medium to long period of time.

2) Depression, anxiety disorder, anthropophobia, a decline in sociality, and adjustment disorder that are suffered by a person who chronically receives various harassments such as power harassment, sexual harassment, and moral harassment over a medium to long period of time.

3) Depression, anxiety disorder, anthropophobia, a reduced sociality, and adjustment disorder that are suffered by a person who chronically receives bullying or harassment at school or company over a medium to long period of time.

4) Depression, anxiety disorder, anthropophobia, a reduced sociality, and adjustment disorder that are suffered by a person who cannot cope with a change in the environment.

5) Anhedonia caused by the above-described diseases.

6) Polydipsia (due to thirst) and polyphagia caused by the above-described diseases.

7) Depression, anxiety disorder, anthropophobia, a decline in sociality, and adjustment disorder that are caused by social defeat stress in a situation in which a person who feels defeated by aggressive actions of a powerful person, but is unable to eliminate or reduce the current sense of defeat from the social standpoints such as companionship at workplace, home, and neighborhood.

Furthermore, it is preferable that the anti-stress composition of the technology is administered to the following subjects.

1) A person having a personality that is considered to be susceptible to depression, such as being punctilious, being serious, having a strong sense of responsibility, working hard, having a strong sense of justice, being unable to refuse when asked, disliking conflicts with people, or always minding people's expectations.
2) A person who is vulnerable to a change in the environment.
3) A person who is highly sensitive to stresses.
4) A person who is in a situation in which the person feels defeated by aggressive actions of a powerful person, but is unable to eliminate or reduce the current sense of defeat from the social standpoints such as companionship at workplace, home, and neighborhood.

Meanwhile, the "powerful person" according to the technology is preferably a person who has relatively higher social status or has relatively greater physical strength than the person who is subjected to stress. Furthermore, the "sense of defeat" according to the technology means that a person feels that his or her power is weak, or considers himself or herself incompetent or worthless, or the like.

The content or the amount of use of *Lactobacillus helveticus* of the technology can be freely set depending on the age, symptom, and the like as long as the efficacy of the technology is not impaired.

In the technology, it is preferable that the amount of *Lactobacillus helveticus* is $2\times10^4$ CFU/kg of body weight/day or more, or $2\times10^4$ cells/kg of body weight/day or more. Meanwhile, the unit "CFU" represents "colony forming unit", and the unit "cells" represents the number of cells (including killed bacteria).

The interval of use (specifically, the interval of administration or ingestion) of *Lactobacillus helveticus* of the technology is not particularly limited, and may be once a day or may be divided into several times a day. Furthermore, it is desirable that the technology is used (specifically, administered or ingested) continuously every day.

Since *Lactobacillus helveticus* that is used for the technology is empirically highly safe, it can be continuously ingested for a long period of time.

<Pharmaceutical composition>

The technology can provide a pharmaceutical composition including *Lactobacillus helveticus* as an active ingredient. The pharmaceutical composition of the technology can be applied to use applications for the prevention, reduction, or suppression of stresses. Specifically, the pharmaceutical composition is an anti-stress medicine or the like.

The pharmaceutical composition of the technology is not particularly limited as long as the pharmaceutical composition contains the *Lactobacillus helveticus.* Regarding the pharmaceutical composition of the technology, the *Lactobacillus helveticus* may be used directly, or may be used after being mixed with a physiologically acceptable liquid or solid preparation carrier and formulated.

The pharmaceutical composition of the technology may be for oral use. Even if *Lactobacillus helveticus* of the technology is administered continuously for a long period of time, adverse side effects are not likely to occur, and therefore, *Lactobacillus helveticus* can be administered without worries.

The dosage form of the pharmaceutical composition of the technology is not particularly limited; however, in the case of oral administration, the pharmaceutical composition can be formulated into, for example, solid preparations such as a powder, a granular preparation, a tablet, a troche, and a capsule, liquid preparations such as a solution, a syrup, a suspension, and an emulsion, and the like. In the case of parenteral administration, the pharmaceutical composition can be formulated into, for example, a suppository, a spray, an inhaler, an ointment, a transdermal patch, an injectable preparation, or the like. Furthermore, on the occasion of formulation, additives such as an excipient, a binder, a disintegrant, a lubricating agent, a stabilizer, a corrigent, a diluent, a surfactant, or a solvent for injection, all of which are conventionally used as preparation carriers, can be used. These components may be appropriately selected by those ordinarily skilled in the art, depending on the formulation.

The content of *Lactobacillus helveticus* of the technology in the pharmaceutical composition of the technology may be appropriately set according to the formulation, usage, the age and gender of the patient, the type of disease, the degree of disease, other conditions, and the like. The content of *Lactobacillus helveticus* of the technology in the pharmaceutical composition of the technology is usually $1\times10^6$ to $1\times10^{12}$ CFU/g or $1\times10^6$ to $1\times10^{12}$ CFU/mL, and preferably in the range of $1\times10^6$ to $1\times10^{12}$ cells/g or $1\times10^6$ to $1\times10^{12}$ cells/mL. In a case in which *Lactobacillus helveticus* of the technology is in the form of killed bacteria, the unit CFU/g or CFU/mL can be replaced with cells/g or cells/mL.

Regarding the timing for administration of the pharmaceutical composition of the technology, the timing for administration can be appropriately selected according to the method for treating a symptom or a disease as an object. Furthermore, the pharmaceutical composition of the technology may be preventively administered, or may be used as a maintenance therapy. The pharmaceutical composition of the technology may be administered once a day, or may be administered in several divided portions a day. Furthermore, it is also acceptable that the pharmaceutical composition of the technology is administered once in several days or several weeks.

<Food or drink composition>

The technology can also provide a food or drink composition including *Lactobacillus helveticus* as an active ingredient. The food or drink composition of the technology can be used in applications for the prevention, reduction, or suppression of stresses. Specifically, the food or drink composition is a health food, a functional food, or the like under the concept of anti-stress applications and the like.

The food or drink composition of the technology is not particularly limited as long as the food or drink composition contains the above-described *Lactobacillus helveticus.* Examples of the food or drink composition include beverages such as soft drinks, carbonated drinks, nutritional drinks, fruit juice drinks, and lactic acid bacteria beverages (including concentrated stock liquids and powders for preparation of these beverages) ; ice creams such as ice cream, sherbet, and snow cone; confectioneries such as toffee, chewing gum, candy, gum, chocolate, tablet candy, snack food, biscuit, jelly, jam, cream, and baked confectionery; dairy products such as processed milk, milk drink, fermented milk, drinking yogurt, and butter; breads; liquid foods such as enteral nutritional food and porridge, weaning food, child-rearing milk, sports drinks; and other functional foods. Furthermore, the food or drink composition of the technology may be a supplement, and may be, for example, a tablet-form supplement. In a case in which the food or drink composition is a supplement, *Lactobacillus helveticus* of the technology can be ingested without being affected by other foods with regard to the dietary intake and consumed calories per day.

Examples of commercially available foods other than those described above include baby foods, furikake condiment, and dried seasoning for rice with tea. The food or drink composition of the technology can be produced by adding the present bacterial cells to a raw material of a conventional food or drink, and the food or drink composition can be produced similarly to a conventional food or drink, except for adding the present bacterial cells. Addition of the present bacterial cells may be carried out at any stage of the production process for the food or drink composition. Furthermore, it is also acceptable that the food or drink composition is produced by going through a fermentation process using the present bacterial cells added thereto. Examples of such a food or drink composition include lactic acid bacteria beverages and fermented milk.

Furthermore, in the food or drink composition of the technology, unless the effects of the technology are not impaired, a component having a probiotics effects or a component assisting a probiotics effects, both of which are known or will be found in the future, can be used. Here, generally, probiotics refer to bacteria having beneficial functions in the intestine. On the other hand, substances that serve as selective nutrition sources for bacteria having beneficial functions in the intestine and promote proliferation of those bacteria are referred to as prebiotics.

For example, the food or drink composition of the technology can be produced by mixing components, including various proteins such as whey protein, casein protein, soy protein, or pea protein (pea protein), or mixtures and degradation products thereof; amino acids such as leucine, valine, isoleucine, or glutamine; vitamins such as vitamin B6 or vitamin C; creatine; citric acid; fish oil; or oligosaccharides such as isomaltooligosaccharide, galactooligosaccharide, xylooligosaccharide, soybean oligosaccharide, fructooligosaccharide, lactulose, and human milk oligosaccharide (HMO), which are prebiotics, with the present bacterial cells. Regarding the human milk oligosaccharides that can be used for the technology, neutral human milk oligosaccharides such as 2'-fucosyllactose, 3-fucosyllactoe, 2',3-difucosyllactose, lacto-N-triose II, lacto-N-tetraose, lacto-N-neotetraose, lacto-N-fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose, and lacto-N-neohexaose; and acidic human milk oligosaccharides such as 3'-sialyllactose, 6'-sialyllactose, 3-fucosyl-3'-sialyllactose, and disialyllacto-N-tetraose, can be used.

The food or drink as defined in the technology can be supplied and sold as a food or drink with a health use application indicated thereon.

The act of "indication" includes all the acts intended for publicizing the above-described application to consumers, and as long as the indication is an expression which may allow recall and analogy of the application, such expressions all correspond to the act of "indication" of the technology, irrespective of the purpose of indication, the content of indication, the object or medium of indication, and the like.

Furthermore, it is preferable that the "indication" is implemented by an expression from which consumers can directly recognize the above-described application. Specifically, acts of transferring, handing over, displaying for transfer or handover, or importing an article of commerce related to a food or drink, the article of commerce or the packaging of the article of commerce having the application described thereon; acts of describing and displaying, or distributing, the application on an advertisement, a price tag, or a transaction document related to the art of commerce, or describing the application on the information based on these as the contents and providing the information by an electromagnetic (the Internet or the like) method; and the like may be mentioned.

On the other hand, regarding the content of the indication, the indication is preferably an indication approved by the government or the like (for example, an indication or the like approved under various institutions established by the government and put in a manner based on the approval). Furthermore, it is preferable that the content of such an indication is attached to a packaging, a container, a catalog, a pamphlet, an advertising material at a selling site, such as POP, to documents other than those, or the like.

Furthermore, examples of the "indication" also include indications that the commodity is a health food, a functional food, an enteral nutritional food, a food for special use, a health functional food, a special health food, a nourishing functional food, a functionally indicated food, a quasi-drug, or the like. Particularly among these, indications approved by Consumers Affairs Agency, for example, indications approved by the institutions related to special health foods, nutritional functional foods, or functionality-indicated foods, or institutions similar to these, or the like may be mentioned. Specific examples include an indication that the commodity is a special health food, an indication that the commodity is a conditional special health food, an indication to the effect that the commodity may affect the structure or function of the body, an indication regarding a reduction of a disease risk, and an indication of the functionality based on a scientific basis. More specifically, typical examples include an indication that the commodity is a special health food (particularly an indication for health use application) defined in Cabinet Office Ordinance on Permitting Special Use Labeling and the like as stipulated in the Health Promotion Act (August 31, 2009, Cabinet Office Ordinance No. 57), and indications similar to this.

Meanwhile, the wording used to implement indications such as described above is not limited to only wordings for the treatment and/or prevention of stress and the like, and it is needless to say that even wordings other than that are also included in the scope of the technology as long as the wordings are wordings indicating the effects of the treatment and/or prevention of various diseases and symptoms related to the treatment and/or prevention of stress. Regarding such wordings, for example, indications based on various use applications for causing consumers to recognize anti-stress effects, such as "for those who are tired from work", "for those who feel stressed from work", and "for those who are worried about interpersonal relations", are also possibly included.

The amount of *Lactobacillus helveticus* of the technology in the food or drink composition of the technology is appropriately set depending on the form of the food or drink composition; however, usually, it is preferable that the amount is in the range of $1 \times 10^6$ to $1 \times 10^{12}$ CFU/g or $1 \times 10^6$ to $1 \times 10^{12}$ CFU/mL, or $1 \times 10^6$ to $1 \times 10^{12}$ cells/g or $1 \times 10^6$ to $1 \times 10^{12}$ cells/mL, in the food or drink.

The food or drink composition of the technology can be produced by adding *Lactobacillus helveticus* of the technology to a raw material of a food or drink composition. The food or drink composition of the technology can be produced by a method similar to the methods for conventional food or drink compositions, except that a step of adding *Lactobacillus helveticus* of the technology is added.

Regarding the raw material for a food or a beverage, any raw material that is used for conventional beverages and foods can be used.

Furthermore, *Lactobacillus helveticus* of the technology can also be used in combination with other lactic acid bacteria that are used in foods and drinks.

In the food or drink composition of the technology, a raw material for the production of a food or drink composition, and materials that are added to foods and drinks during the production process or after production of a food or drink composition, such as food additives, are also included.

In the production of the food or drink composition of the technology, the addition of *Lactobacillus helveticus* of the technology may be carried out at any stage of a production process for the food or drink composition. For example, *Lactobacillus helveticus* of the technology may be added generally in a step of adding a lactic acid bacterium as an example.

The food or drink composition of the technology may be powdered milk, and this can be used particularly for formulated milk powder for infants, and milk for mothers directed at mothers during pregnancy and lactation. Formulated milk powder for infants refers to formulated milk powder for infants directed at 0- to 12-month old infants, follow-up formula milk directed at 6- to 9-month old infants and young children (up to 3 years old), formulated milk powder for low birth weight infants directed at newborn babies having a body weight at birth of less than 2,500 g (low birth weight infants), and various therapeutic milks used for the treatment of infants having pathological conditions such as milk allergy and lactose intolerance. Furthermore, this composition can be applied to health functional foods and foods for patients. The institutions for health functional foods were established aiming at not only ordinary foods but also for foods in the form of tablets, capsules, and the like, based on the internal and external trends and the consistency with conventional institutions for special health foods. The institutions are composed of two types, namely, institutions for special health foods (individual permission type) and nutritional functional foods (standards and criteria type).

Examples of the food or drink according to the technology include milk for mothers (formulated milk powder) and nutritional supplement foods exclusive for mothers during pregnancy and lactation. Milk for mothers refers to milk in which nutrients necessary for pregnancy and lactation have been incorporated in a well-balanced manner.

The formulated milk powder of the technology may be specifically, for example, anti-stress powdered milk. The anti-stress powdered milk can be produced by, for example, the following method.

That is, the technology provides a method for producing anti-stress powdered milk, the method including the following steps (A) to (C):

(A) a step of culturing *Lactobacillus helveticus* bacterium in a medium containing a milk component, and obtaining a culture product;
(B) a step of subjecting the culture product to spray drying and/or freeze-drying, and obtaining a bacterial cell powder; and
(C) a step of mixing the bacterial cell powder with prebiotics, and obtaining anti-stress powdered milk.

Here, the milk component may be mixed simultaneously with the step (C), before the step (C), or after the step (C).

Furthermore, the technology provides a method for producing anti-stress powdered milk, the method including the following step (A):

(A) a step of mixing prebiotics, *Lactobacillus helveticus* bacterium, and a milk component, and obtaining powdered milk.

Examples of the milk component include cow milk, buffalo milk, sheep milk, goat milk, horse milk, skim milk, concentrated skimmed milk, skim milk powder, concentrated milk, whole milk powder, cream, butter, buttermilk, condensed milk, and milk protein. Furthermore, as the milk protein, for example, whey, casein, and the like and hydrolysates of these or the like can be used. Meanwhile, the hydrolysates can be produced by hydrolyzing whey, casein, and the like using an acid, an alkali, an enzyme (for example, a proteolytic enzyme), or the like.

Furthermore, the food composition of the technology may be, for example, an anti-stress supplement. An anti-stress supplement can be produced by, for example, the following method.

That is, the technology provides a method for producing an anti-stress supplement, the method including the following steps (A) and (B) :

(A) a step of mixing *Lactobacillus helveticus* bacterium and an excipient, and obtaining a mixture; and
(B) a step of tableting the mixture.

For both of the production methods described above, components other than the components mentioned in the above-described steps may be used appropriately in combination.

Meanwhile, the technology can also employ the following configurations.

[1] An anti-stress composition comprising *Lactobacillus helveticus* of the technology as an active ingredient.
[2] A method for prevention, reduction, or suppression of stress, the method comprising administering or ingesting *Lactobacillus helveticus* of the technology.
[3] *Lactobacillus helveticus* used for prevention, reduction, or suppression of stress, or use thereof.
[4] Use of *Lactobacillus helveticus* for producing an anti-stress composition.
[5] Use of *Lactobacillus helveticus* in a stress relieving agent, a medicine for stress relief, or a food or drink for stress relief.
[6] A method for relieving stress, the method including administering or ingesting *Lactobacillus helveticus.*
[7] A method for relieving stress, the method including administering *Lactobacillus helveticus* to a person who is highly sensitive to stress.
[8] Use of *Lactobacillus helveticus* for treatment or prevention of anxiety disorder, anthropophobia, a decline in sociality, or adjustment disorder, all of which occur under stress.
[9] Amethod for treatment or prevention of anxiety disorder, anthropophobia, a decline in sociality, or adjustment disorder, all of which occur under stress, the method comprising administering or ingesting *Lactobacillus helveticus.*
[10] Suitably, in any one of the above items [1] to [9], the stress is mental stress.
[11] Suitably, in any one of the above items [1] to [10], the *Lactobacillus helveticus* is *Lactobacillus helveticus* NITE BP-01671.
[12] Suitably, in any one of the above items [1] to [11], the technology is intended for a medicine, or for a food or drink.
[13] A method for producing an anti-stress composition, the method including the following steps (A) to (C):

(A) a step of culturing *Lactobacillus helveticus* in a medium containing a milk component, and obtaining a culture product;
(B) a step of subjecting the culture product to spray drying and/or freeze-drying, and obtaining a bacterial cell powder; and

(C) a step of mixing the bacterial cell powder with prebiotics, and obtaining an anti-stress composition.

[14] A method for producing an anti-stress composition, the method including the following step (A):

(A) a step of mixing prebiotics, *Lactobacillus helveticus* bacterium, and a milk component, and obtaining an anti-stress composition.

[15] The method according to the above item [13] or [14], in which the milk component is milk protein.
[16] The method according to the above item [15], in which the milk protein is at least one component selected from the group consisting of whey, whey hydrolysate, and casein.
[17] A method for producing an anti-stress supplement, the method including the following steps (A) and (B):

(A) a step of mixing a bacterium of the genus Lactobacillus helveticus and an excipient, and obtaining a mixture; and
(B) a step of tableting the mixture.

Examples

Hereinafter, the technology will be described in more detail based on Examples. Meanwhile, the Examples that will be described below represent examples of representative Examples of the technology, and the scope of the technology should not be construed to be narrow by these Examples.

[Experimental Example 1]

(1) Experiment method

In the present experiment, an experiment of causing a small mouse to experience social defeat by allowing the small mouse to be bitten many times by a large mouse was carried out, and an effect of *Lactobacillus helveticus* on behavioral changes caused by social defeat stress (decrease in escape behavior, a lethargic state) was investigated.
In the present experiment, C57BL/6J mice were used as social defeat stress-bearing mice. In a cage of male and female ICR mice cohabiting for three weeks or longer, only the male parent was left. A C57BL/6J mouse was brought into that cage, and the time taken by the C57BL/6J mouse to be bitten 20 times by the male parent mouse (escape time) was measured. As the escape time increases, it is implied that the C57BL/6J mouse attempts to escape from the ICR mouse, and therefore, it was determined that the social defeat stress is reduced.
From two days before the beginning of giving social defeat stress until the end of the behavioral test, the experiment was carried out respectively for mice to which milnacipran was administered (Toledomin tablets; manufactured by Janssen Pharmaceutical K.K.) (positive control), mice to which nothing was administered (vehicle control), and mice to which living bacterial cells of *Lactobacillus helveticus* NITE BP-01671 were orally administered once a day using a sonde needle.

(2) Experimental results

The results of the present experiment are presented in the following Table 1.

[Table 1]

[Table 1]

|  | Day 1 | | Day 2 | | Day 3 | | Day 4 | | Day 5 | |
|---|---|---|---|---|---|---|---|---|---|---|
|  | Average value | S.E. | Average value | S.E. | Average value | S.E. | Average value | S.E. | Average value | S.E. |
| Positive control: milnacipran | 24.6 | 4.5 | 42.9 | 11.0 | 65.9 | 18.6 | 63.2 | 21.9 | 81.0 | 41.7 |
| Vehicle control | 27.8 | 3.0 | 23.8 | 2.6 | 23.0 | 2.5 | 17.6 | 1.3 | 18.7 | 1.3 |
| L. helveticus NITE BP-01671 | 31.8 | 8.6 | 40.7 | 17.5 | 25.6 | 4.3 | 32.0 | 7.6 | 99.7 | 49.5 |

|  | Day 8 | | Day 9 | | Day 10 | | Day 11 | | Day 12 | |
|---|---|---|---|---|---|---|---|---|---|---|
|  | Average value | S.E. | Average value | S.E. | Average value | S.E. | Average value | S.E. | Average value | S.E. |
| Positive control: milnacipran | 114.8 | 58.4 | 159.0 | 44.4 ** | 183.2 | 67.1 * | 183.9 | 63.8 * | 143.2 | 40.2 ** |
| Vehicle control | 16.4 | 1.5 | 17.6 | 1.6 | 18.8 | 1.8 | 17.0 | 0.9 | 16.8 | 1.8 |
| L. helveticus NITE BP-01671 | 58.5 | 20.7 | 103.2 | 56.6 | 95.2 | 35.7 | 147.0 | 59.2 | 138.4 | 51.9 |

|  | Day 15 | | Day 16 | |
|---|---|---|---|---|
|  | Average value | S.E. | Average value | S.E. |
| Positive control: milnacipran | 68.7 | 17.9 ** | 46.9 | 7.9 ** |
| Vehicle control | 16.6 | 1.3 | 18.5 | 2.8 |
| L. helveticus NITE BP-01671 | 89.7 | 35.5 | 68.7 | 31.9 |

*$p < 0.05$, **$p < 0.01$ There is a significant difference with respect to the vehicle control (student's t-test)

#$p < 0.05$ There is a significant difference with respect to the vehicle control (student's t-test)

As shown in Table 1, in the vehicle control, the escape time was reduced to about 17 seconds due to social defeat stress after the fourth day; however, in the mice to which milnacipran was administered, the escape time increased significantly compared to the vehicle control. Furthermore, the mice to which *Lactobacillus helveticus* NITE BP-01671 was administered also showed a behavior similar to that of the mice to which milnacipran was administered. Therefore, it was recognized that an effect of reducing social defeat stress is obtained by ingesting *Lactobacillus helveticus* NITE BP-01671.

[Experimental Example 2]

(1) Experiment method

In the present experiment, an experiment of causing a small mouse to repeatedly experience chronic social defeat by being attacked by a large mouse every day was carried out, and an effect of *Lactobacillus helveticus* on behavioral changes (loss of pleasant feeling) caused by exposure to social defeat stress for several times was investigated.

In the present experiment, C57BL/6J mice were used as social defeat stress-bearing mice, and heat-killed bacterial cells of *Lactobacillus helveticus* NITE BP-01671 were administered from two days before the beginning of giving social defeat stress until the end of the behavioral test. Culture of *Lactobacillus helveticus* NITE BP-01671 was carried out by liquid stationary culture in MRS medium at 37°C for 16 hours. After the bacterial cells were collected in a centrifuge, the bacterial cells were suspended in physiological saline and heated at 90°C for 30 minutes, and thereby heat-killed bacterial cells were obtained. The heat-killed bacterial cells were administered to mice by mixing the cells into the mouse diet (AIN-93G) . A C57BL/6J mouse was brought into the territory of an ICR mouse, and the mice were allowed to fight for 5 minutes. Subsequently, the two were transferred into an SD cage obtained by installing a partition plate in an M cage (manufactured by Natsume Seisakusho Co., Ltd.), and the mice were reared for 24 hours in the same cage. This rearing was continued for 9 days. Two days thereafter, one bottle containing distilled water and one bottle containing 1% sucrose water were installed in the cage of the mice, and the amounts of water intake until the next day were respectively measured. Thereby, the ratio of the amount of ingestion of 1% sucrose water to the amount of ingestion of ordinary water (hereinafter, referred to as ingestion ratio for sucrose water) was calculated. Mice have a habit of ingesting sucrose water with preference; however, a mouse subjected to social defeat stress loses a pleasant feeling and turns to dislike sweet water. Thus, it was thought that when a decrease in the ingestion ratio for sucrose water was suppressed, the loss of a pleasant feeling was alleviated, and it was considered that the social defeat stress was reduced.

(2) Experiment results

The results of the present experiment are presented in the following Table 2.

[Table 2]

[Table 2]

| | Normal group (free of stress) | control group (under stress, normal diet) | *Lactobacillus helveticus* NITE BP-01671 administered group (under stress, diet including heat-killed bacterial cells) |
|---|---|---|---|
| Average value | 302.3 | 131.6 | 234.2 |
| Standard error | 41.8 | 22.0 | 41.1 |

As shown in Table 2, compared to a normal group that was not subjected to stress, the ingestion ratio for sucrose water was decreased in a control group that was subjected to stress. On the other hand, in a *Lactobacillus helveticus* NITE BP-01671 administered group, , a decrease in the ingestion ratio for sucrose water was suppressed compared to the control group. Therefore, it was recognized that an effect of reducing social defeat stress is obtained by ingesting heat-killed bacterial cells of *Lactobacillus helveticus* NITE BP-01671.

[Experimental Example 3]

(1) Experiment method

In the present experiment, an experiment of causing a small mouse to repeatedly experience chronic social defeat by being attacked by a large mouse every day was carried out, and an effect of *Lactobacillus helveticus* on behavioral changes (loss of sociality) caused by exposure to social defeat stress for several times was investigated.

In the present experiment, C57BL/6J mice were used as social defeat stress-bearing mice, and heat-killed bacterial cells of *Lactobacillus helveticus* NITE BP-01671 were administered from two days before the beginning of giving social defeat stress until the end of the behavioral test. Meanwhile, regarding the heat-killed bacterial cells, the same cells as those of [Experimental Example 2] were used. The heat-killed bacterial cells were administered to mice by mixing the cells into the mouse diet (AIN-93G) . A C57BL/6J mouse was brought into the territory of an ICR mouse, and the mice were allowed to fight for 5 minutes. Subsequently, the two were transferred into an SD cage obtained by installing a partition plate in an M cage (manufactured by Natsume Seisakusho Co., Ltd.), and the mice were reared for 24 hours in the same cage. This rearing was continued for 9 days. Two days thereafter, a C57BL/6J mouse was introduced into the case of an ICR mouse, and sociality was investigated from the degree of contact thereof. An interaction zone (area having a width of 10 cm surrounding the place where the ICR mouse was placed) was determined in advance in the periphery of the area where the ICR mouse was placed, the time in which the C57BL/6J mouse stayed in the interaction zone was measured, and thereby the change in sociality of the C57BL/6J mouse was measured. Meanwhile, the time in which the mouse stayed in the interaction zone was analyzed using a CCD camera with an automated tracing system (Time OFCR1 software; O'Hara & Co., Tokyo, Japan).

(2) Experiment results

The results of the present experiment are presented in the following Table 3. Meanwhile, the values shown in the table were values obtained by the following formula.

$$100 \times [\text{Time in which C57BL/6J mouse stayed in the interaction zone (with ICR mouse)}]/[\text{time in which C57BL/6J mouse stayed out of the interaction zone (without ICR mouse)}]$$

[Table 3]

[Table 3]

|  | Normal group (free of stress) | control group (under stress,normal diet) | *Lactobacillus helveticus* NITE BP-01671 administered group (under stress, diet including heat-killed bacterial cells) |
|---|---|---|---|
| Average value | 174.3 | 64.4 | 135.6 |
| Standard error | 34.2 | 11.8 | 30.2 |

As shown in Table 3, compared to a normal group that was not subjected to stress, the time in which the mice stayed in the interaction zone decreased in a control group that was subjected to stress. That is, sociality was deteriorated. On the other hand, in *Lactobacillus helveticus* NITE BP-01671 administered group,, the time in which the mice stayed in the interaction zone increased, compared to the control group. That is, a decrease in sociality was suppressed. Therefore, it was recognized that an effect of reducing social defeat stress is obtained by ingesting heat-killed bacterial cells of *Lactobacillus helveticus* NITE BP-01671.

[Production Example 1: Fermented milk]

1,000 mL of 10% (W/W) reduced skim milk powder medium obtained by dissolving 10% (W/W) reduced skim milk powder (manufactured by MORINAGA MILK INDUSTRY CO., LTD.) in water is sterilized at 90°C for 30 minutes, 30 mL of a seed culture of *Lactobacillus helveticus* NITE BP-01671 is inoculated therein, and culture is carried out for 16 hours at 37°C.

Apart from this, 50 L of a base containing 3.0% (W/W) of milk fat and 9.0% (W/W) of non-fat milk solid content is heated to 70°C and is homogenized at a pressure of 15 MPa, and then the base is sterilized at 90°C for 10 minutes and cooled to 40°C. Into this sterilized base, 500 mL of a culture of *Lactobacillus helveticus* NITE BP-01671 that has been subjected to pre-culture as described above is inoculated, and a resin container having a volume of 500 mL is filled with the mixture and is sealed. Culture is performed for 7 hours at 37°C, and then the mixture is immediately cooled. Thereby, an anti-stress composition (fermented milk) containing *Lactobacillus helveticus* NITE BP-01671 can be obtained. When this fermented milk is ingested such that the amount of ingestion of *Lactobacillus helveticus* per day is $2\times10^4$ CFU/kg of body weight/day or more, an anti-stress effect can be expected.

[Production Example 2: Powder preparation]

A reduced skim milk powder medium (containing 13% by weight of skim milk powder and 0.5% by weight of yeast extract) is sterilized for 30 minutes at 95°C, subsequently a seed culture of *Lactobacillus helveticus* NITE BP-01671 is inoculated into the medium, and culture is performed for 16 hours at 37°C. A culture product thus obtained is freeze-dried, and a powder preparation of a culture product of *Lactobacillus helveticus* NITE BP-01671 can be obtained. When this powder preparation is administered such that the amount of administration of *Lactobacillus helveticus* per day is $2\times10^4$ CFU/kg of body weight/day or more, an anti-stress effect can be expected.

[Production Example 3: Formulated milk powder]

10 kg of a desalted milk whey protein powder (manufactured by Milei GmbH), 6 kg of a milk casein powder (manufactured by Fonterra Co-operative Group, Ltd.), 48 kg of lactose (manufactured by Milei GmbH), 920 g of a mineral mixture (manufactured by Tomita Pharmaceutical Co., Ltd.), 32 g of a vitamin mixture (manufactured by Mitsubishi Tanabe Pharma Corporation), 500 g of lactulose (manufactured by MORINAGA MILK INDUSTRY CO., LTD.), 500 g of raffinose (manufactured by Nippon Beet Sugar Manufacturing Co., Ltd.), and 900 g of galactooligosaccharide liquid sugar (manufactured by Yakult Pharmaceutical Industry Co., Ltd.) were dissolved in 300 kg of warm water, and the solution was further heated and dissolved for 10 minutes at 90°C. 28 kg of formulated fats (manufactured by TAIYO YUSHI CORP.) was added thereto, and the mixture was homogenized. Subsequently, sterilization and concentration processes were performed, the mixture was spray dried, and thus about 95 kg of a formulated milk powder was prepared. To this, 100 g of a bacterial cell powder of *Lactobacillus helveticus* NITE BP-01671 ($1.8\times10^{11}$ cfu/g, manufactured by MORINAGA MILK INDUSTRY CO., LTD.) triturated in starch is added, and about 95 kg of a bifidobacterium- and oligosaccharide-incorporated formulated milk powder is prepared. When the formulated milk powder thus obtained is dissolved in water, and thereby a formulated milk liquid having a total solid content concentration of 14% (w/V), which is a standard milk formula concentration, is obtained, the number of bifidobacterial cells in the formulated milk liquid that can be obtained is $2.7\times10^9$ cfu/100 mL. By administering a modified milk powder obtained as described above, an anti-stress effect can be expected.

Industrial Applicability

The anti-stress composition of the technology is such that since an active ingredient is *Lactobacillus helveticus,* the anti-stress composition is excellent in safety and has fewer adverse side effects even when administered or ingested continuously for a long period of time. Therefore, the anti-stress composition is very useful.

Deposit Number

**[0090]** *(1) Lactobacillus helveticus* NITE BP-01671 (Deposit No.: NITE BP-01671, Date of deposit: July 29, 2013), Depositary: 2-5-8 Kazusakamatari, Kisarazu-shi, Chiba Prefecture, 292-0818, Japan, National Institute of Technology and Evaluation Patent Microorganisms Depositary (NPMD).

**Claims**

1. An anti-stress composition, comprising *Lactobacillus helveticus* as an active ingredient.

2. The composition according to claim 1, wherein the stress is mental stress.

3. The composition according to claim 1 or 2, wherein the composition is used for treatment or preventions of anxiety disorder, anthropophobia, a reduced sociality, or adjustment disorder.

4. The composition according to any one of claims 1 to 3, wherein the *Lactobacillus helveticus* is *Lactobacillus helveticus* NITE BP-01671.

5. The composition according to any one of claims 1 to 4, wherein the composition is a pharmaceutical composition or a food or drink composition.

6. A method for prevention, reduction, or suppression of stress, the method comprising administering or ingesting *Lactobacillus helveticus.*

7. *Lactobacillus helveticus* used for prevention, reduction, or suppression of stress.

8. Use of *Lactobacillus helveticus* for producing an anti-stress composition.

9. Use of *Lactobacillus helveticus* in a stress reducing composition, a pharmaceutical for stress reducing, or a food or drink for stress relief.

10. A method for reducing stress, the method comprising administering or ingesting *Lactobacillus helveticus.*

11. A method for relieving stress, the method comprising administering *Lactobacillus helveticus* to a person who is highly sensitive to stress.

12. Use of *Lactobacillus helveticus* for treatment and/or prevention of anxiety disorder, anthropophobia, a decline in sociality, or adjustment disorder, all of which occur under stress.

13. A method for treatment and/or prevention of anxiety disorder, anthropophobia, a decline in sociality, or adjustment disorder, all of which occur under stress, the method comprising administering *Lactobacillus helveticus* to a subject.

## INTERNATIONAL SEARCH REPORT

| International application No. |
|---|
| PCT/JP2019/012303 |

### A. CLASSIFICATION OF SUBJECT MATTER
Int. Cl. A61K35/747(2015.01)i, A23L33/135(2016.01)i, A61P25/00(2006.01)i, C12N1/20(2006.01)i

According to International Patent Classification (IPC) or to both national classification and IPC

### B. FIELDS SEARCHED

Minimum documentation searched (classification system followed by classification symbols)
Int. Cl. A61K35/747, A23L33/135, A61P25/00, C12N1/20

Documentation searched other than minimum documentation to the extent that such documents are included in the fields searched
Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)
JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X<br>Y | LIANG, S. et al., Administration of Lactobacillus helveticus NS8 improves behavioral, cognitive, and biochemical aberrations caused by chronic restraint stress. Neuroscience., 2015, vol. 310, pp. 561-77. (doi:10.1016/j.neuroscience.2015.09.033.) (abstract, introduction, fig. 3, 4, pp. 565, 573) | 1-3, 5-13<br>4, 5 |
| X<br>Y | WANG, H. et al., Effect of Probiotics on Central Nervous System Functions in Animals and Humans A Systematic Review. J Neurogastroenterol Motil., 2016, vol. 22, no. 4, pp. 589-605, (doi:10.5056/jnm 16018.) (p. 596 anxiety, table 1, pp. 593, 594, table 2, p. 599, 596 stress response) | 1-3, 5-13<br>4, 5 |

☒ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 30.05.2019 | 18.06.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2019/012303 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2015-047114 A (MORINAGA MILK INDUSTRY CO., LTD.) 16 March 2015, entire text (Family: none) | 4, 5 |
| A | LUO, J. et al., Ingestion of Lactobacillus strain reduces anxiety and improves cognitive function in the hyperammonemia rat. Sci China Life Sci., 2014, vol. 57, no. 3, pp. 327-335. (doi:10. 1007/s 1 1427-014-4615-4.) entire text | 1-13 |
| A | JP 1998-045610 A (CALPIS CO., LTD.) 17 February 1998, entire text & US 6596301 B1, entire text & EP 966969 A1 & AU 3782897 A & CN 1232400 A & KR 10-2000-0029867 A | 1-13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010030901 A

- JP 2015047114 A

**Non-patent literature cited in the description**

- *National Institute of Technology and Evaluation Patent Microorganisms Depositary,* 29 July 2013

- **AUGUST 31, 2009.** Health Promotion Act. Cabinet Office Ordinance